# EUROPÄISCHE PATENTANMELDUNG

(11) **EP 4 656 082 A1**
(43) Veröffentlichungstag der Anmeldung: **03.12.2025**
(21) Anmeldenummer: 25175686.2
(22) Anmeldetag: 12.05.2025
(51) Int. Cl.: A41B 9/00, A41D 13/12, A61F 13/14

(54) **KOMPRESSIONSHOSE**

(30) Priorität: 29.05.2024 DE 102024115125
(71) Anmelder: Julius Zorn GmbH, 86551 Aichach (DE)
(72) Erfinder: Prof. Dr. Taeger, Christian, 81479 München (DE); Leitloff, Mathias, 95463 Bindlach (DE)
(74) Vertreter: Charrier Rapp & Liebau

(57) **Zusammenfassung**

Die Erfindung betrifft eine Kompressionshose zur kompressiven Behandlung von Ödemen oder anderer Erkrankungen oder Verletzungen im Genital- oder Beckenbereich eines Patienten, umfassend ein Taillenband (1) mit ersten Befestigungsmitteln (2), ein Hosenteil (3) und ein mit zweiten Befestigungsmitteln (4) ausgestattetes Kompressionselement (5) zur Ausübung einer Kompression auf den Genitalbereich des Patienten, wobei das Taillenband (1) mittels Verschlusselemente (6) verstellbar im Bereich der Taille des Patienten anlegbar ist, wobei das Kompressionselement (5) mittels einer lösbaren Kopplung der ersten Befestigungsmittel (2) und der zweiten Befestigungsmittel (4) an dem Taillenband (1) befestigbar ist. Um die Einstellung eines individuell und präzise anpassbaren Kompressionsdrucks und ein bequemes Tragen zu ermöglichen, ist vorgesehen, dass das Taillenband (1) und das Hosenteil (3) voneinander getrennte oder voneinander trennbare Teile der Kompressionshose sind und dass das Kompressionselement (5) als einteiliger Beutel oder als einteilige Schürze aus einem elastischen Textilmaterial ausgebildet und an dem Hosenteil (3) befestigt ist.

## Beschreibung

Die Erfindung betrifft eine Kompressionshose, insbesondere zur kompressiven Behandlung von Ödemen oder anderer Erkrankungen oder Verletzungen im Genital- oder Beckenbereich eines Patienten und zur Unterstützung der postpartalen Rückbildung nach einer Entbindung, nach dem Oberbegriff des Anspruchs 1.

Aus der EP 2 884 862 B1 ist ein medizinisches Kleidungsstück in Form einer Hüft- und Bauchstütze zur Unterstützung des Beckens und des Unterleibs eines Patienten bekannt, mit dem Schmerzen, Ödeme und Infektionen behandelt und kontrolliert werden können, die bei akuten oder chronischen Erkrankungen wie Krebs oder nach genitalen oder abdominalen Operationen oder vaginalen Entbindungen auftreten, um Komplikationen zu verringern und die Genesungszeit zu verbessern. Die Hüft- und Bauchstütze umfasst dabei ein die Taille umgebendes Element, das an seinem vorderen Teil verstellbar befestigt werden kann und einen oberen und einen unteren Rand sowie einen hinteren Teil aufweist, sowie Shorts, die nicht abnehmbar an der Unterkante des die Taille umschließenden Elements befestigt sind und einen Schrittbereich aufweisen, sowie ein Riemenelement, das an einem Ende am hinteren Teil des die Taille umgebenden Elements befestigt ist und dessen anderes Ende nach dem Hindurchführen unter dem Schrittbereich der Shorts am anderen Ende der Vorderseite des die Taille umgebenden Elements über einen Klettverschluss fixierbar ist, sodass das Riemenelement einen Druck auf den Schrittbereich des Trägers ausübt, wobei das Riemenelement aus drei Riemen besteht, sodass der Träger das andere Ende eines der drei Riemen an einem mittleren Teil der Vorderseite des die Taille umgebenden Elements befestigen kann und die anderen Enden der beiden übrigen Riemen seitlich verschoben in Bezug auf das andere Ende des einen Riemens befestigen kann.

Die bekannte Hüft- und Bauchstütze stellt ein einstückiges und individuell einstellbares medizinisches Kleidungsstück dar, das unmittelbar nach einer Operation oder nach einer Entbindung und danach während einer mehrwöchigen Erholungsphase der zu behandelnden Person über der Unterwäsche getragen werden kann, um den Rücken, die Bauchmuskeln und die Vulva zu stützen. Durch die einteilige Ausbildung des medizinischen Kleidungsstücks erweist es sich jedoch als schwierig, dieses an- und auszuziehen. Weiterhin erweist sich das bekannte Kleidungsstück als nachteilig hinsichtlich der Flexibilität und der individuellen Anpassung an verschiedene Patienten mit unterschiedlichen Größenverhältnissen im Bauch- und Hüftbereich. Weitere Nachteile ergeben sich aus der einteiligen Ausbildung des medizinischen Kleidungsstücks in Bezug auf den Tragekomfort, bspw. beim Bücken, weil das die Taille umgebende Element nach unten gezogen wird.

In anderen Anwendungsfällen für medizinische Hüft- und Bauchstützen, wie z.B. der kompressiven Behandlung von Genitalödemen oder anderen Ödemen im Becken- oder Leistenbereich, ist es darüber hinaus vorteilhaft, wenn ein vorgegebener Kompressionsdruck im Hüftbereich erzeugt werden kann. Hierfür ist die bekannte Hüft- und Bauchstütze nicht geeignet. Darüber hinaus erweist sich die bekannte Hüft- und Bauchstütze als unbequem, wenn ein erhöhter Kompressionsdruck auf den Genitalbereich mit den drei Riemen des Riemenelements eingestellt werden soll.

Vor diesem Hintergrund besteht die Aufgabe der Erfindung in der Bereitstellung einer Kompressionshose zur kompressiven Behandlung von Ödemen oder anderer Erkrankungen oder Verletzungen im Genital- oder Beckenbereich eines Patienten oder einer Patientin, die eine individuelle und präzise Anpassung an die Körperform, die Einstellung eines gewünschten, individuell und präzise anpassbaren Kompressionsdrucks sowohl im Genitalbereich und der Leistengegend als auch im Hüftbereich und ein bequemes Tragen über eine längere Behandlungsdauer ermöglicht. Dabei soll der von der Kompressionshose erzeugte Kompressionsdruck auf einfache Weise während der Behandlungsdauer mit einer genauen Justierung des gewünschten Kompressionsdrucks präzise einstellbar und an den Behandlungserfolg anpassbar sein. Weiterhin soll ein einfaches An- und Ausziehen der Kompressionshose ermöglicht werden.

Diese Aufgaben werden mit einer Kompressionshose mit den Merkmalen des Anspruchs 1 gelöst. Bevorzugte Ausführungsformen der Kompressionshose gehen aus den abhängigen Ansprüchen hervor.

Die Kompressionshose dient insbesondere zur kompressiven Behandlung von Ödemen oder anderer Erkrankungen oder Verletzungen im Genital- oder Beckenbereich eines Patienten und kann insbesondere von männlichen und auch von weiblichen Patienten, bspw. zur postpartalen Rückbildung nach einer Entbindung, getragen werden. Die Kompressionshose umfasst ein Taillenband mit ersten Befestigungsmitteln, ein Hosenteil und ein mit zweiten Befestigungsmitteln ausgestattetes Kompressionselement zur Ausübung einer Kompression auf den Genitalbereich des Patienten, wobei das Taillenband mittels Verschlusselemente verstellbar im Bereich der Taille oder im oberen Hüftbereich des Patienten anlegbar ist, wobei die ersten Befestigungsmittel zumindest im oder unterhalb des Bauchbereich des Patienten zu liegen kommen und das Hosenteil im angezogenen Zustand zumindest den Beckenbereich des Patienten vollumfänglich umschließt und das Kompressionselement mittels einer lösbaren Kopplung der ersten Befestigungsmittel und der zweiten Befestigungsmittel an dem Taillenband befestigbar ist. Dabei sind das Taillenband und das Hosenteil voneinander getrennte oder voneinander trennbare Teile der Kompressionshose und das Kompressionselement ist als einteiliger Beutel oder als einteilige Schürze aus einem elastischen Textilmaterial ausgebildet und an dem Hosenteil befestigt.

Durch die getrennte oder trennbare Ausbildung des Taillenbands und des Hosenteils entsteht eine zweiteilige Kompressionshose, die sich leichter und bequemer an- und ausziehen lässt als eine einteilige Kompressionshose. Die lösbare Kopplung der ersten Befestigungsmittel und der zweiten Befestigungsmittel zur Befestigung des Kompressionselements an dem Taillenband ermöglicht eine gezielte und präzise Einstellung eines vom Kompressionselement auf den Genitalbereich bzw. den Leistenbereich des Patienten auszuübenden Kompressionsdrucks. Dabei sind die ersten Befestigungsmittel und die zweiten Befestigungsmittel zweckmäßig so ausgestaltet, dass sie an unterschiedlichen Stellen miteinander gekoppelt werden können, wobei der ausgeübte Kompressionsdruck von der Position, an der die ersten und zweiten Befestigungsmittel miteinander gekoppelt werden, abhängt.

Die ersten Befestigungsmittel und die zweiten Befestigungsmittel sind bevorzugt als jeweils miteinander korrespondierende Bestandteile eines zweiteiligen, reversiblen Verbindungsmittels, insbesondere eines Klettverschlusses, eines Haftverschlusses oder einer Druckknopfverbindung, ausgebildet. Die ersten Befestigungsmittel sind bspw. als flächiges Klettpad ausgebildet, welches auf einer Außenseite des Taillenbands angeordnet ist. Die zweiten Befestigungsmittel können als dazu korrespondierendes Klettband ausgebildet und an einem (freien) Ende des Kompressionselements angeordnet sein. Dadurch können die zweiten Befestigungsmittel (Klettband) an einer beliebigen Stelle am Klettpad der ersten Befestigungsmittel fixiert werden, um einen gewünschten Kompressionsdruck des Kompressionselements einzustellen. Je höher die Fixierposition des Klettbands der zweiten Befestigungsmittel (Klettband) am Klettpad der ersten Befestigungsmittel ist, desto mehr wird das elastische Kompressionselement gedehnt und desto höher ist der vom Kompressionselement auf den Genitalbereich und die Leistengegend des Patienten ausgeübte Kompressionsdruck.

Die ersten Befestigungsmittel sind bevorzugt flächig und insbesondere als flächiges Flauschteil eines Klettverschlusses ausgebildet und auf einer Außenseite eines vorderen Abschnitts des Taillenbands, der bei angelegtem Taillenbands im Bauchbereich des Patienten zu liegen kommt, angeordnet. Dies ermöglicht eine Vielzahl von möglichen Fixierposition des Klettbands der zweiten Befestigungsmittel (Klettband) am Flauschteil (Klettpad) der ersten Befestigungsmittel im Bauchbereich des Patienten und dadurch eine hohe Variation bei der Einstellung eines gewünschten Kompressionsdrucks, der vom Kompressionselement auf den Genitalbereich des Patienten ausgeübt werden soll.

Das Taillenband ist mittels der am Taillenband angeordneten Verschlusselemente im Bereich der Taille oder des oberen Hüftbereichs des Patienten fixierbar. Die Verschlusselemente des Taillenbands sind bevorzugt als lösbare Befestigungselemente, insbesondere als Klettverschluss oder als Gürtelschnalle, ausgebildet und in einem Endabschnitt des Taillenbands angeordnet. Dadurch kann das Taillenband unter leichter Dehnung bequem und gelichzeitig sicher fixiert um den Taillen- oder Hüftumfang des Patienten angelegt werden. Die Verschlusselemente sind dabei bevorzugt im Bauchbereich des Patienten angeordnet, wodurch dem Patienten ein bequemes Anlegen und Fixieren des Taillenbands ermöglicht wird. Durch Festlegen der Verschlusselemente an unterschiedlichen Positionen in einer Umfangsrichtung des Patienten kann ein vom Taillenband auf den Taillenbereich und/oder den Hüftbereich des Patienten ausgeübter Druck eingestellt werden.

Durch die Ausbildung des Kompressionselements als einteiliger Beutel oder als einteilige Schürze aus einem elastischen Textilmaterial wird ein gleichmäßiger, flächiger Kompressionsdruck erzeugt, wenn das Kompressionselement bei am Patienten angelegter Kompressionshose unter Dehnung des Kompressionselements mittels einer Kopplung der ersten und zweiten Befestigungsmittel am Taillenband befestigt ist. Das als Beutel bzw. Schürze ausgeformte Kompressionselement ist dabei zweckmäßig so dimensioniert, dass das Kompressionselement den Genitalbereich des Patienten zumindest weitgehend umschließt.

In einer Ausführungsform der Kompressionshose für Männer ist das beutelförmig ausgebildete Kompressionselement an die Anatomie des Patienten so angepasst, dass die Genitalien vollständig vom Kompressionselement aufgenommen werden können. Dies ermöglicht ein Einfassen eines zu behandelnden Ödems durch das Kompressionselement. Das beutelförmig ausgebildete Kompressionselement wirkt dabei auch als Stützbeutel für die Genitalien.

Die erfindungsgemäße Kompressionshose ermöglicht daher insgesamt eine individuelle und präzise Einstellung eines von der Kompressionshose auf ein Genital-Ödem bzw. auf den Unterbauch und den Hüftbereich des Patienten ausgeübten Kompressionsdrucks sowie einen verbesserten Tragekomfort. Das separat vom Hosenteil anlegbare Taillenband ermöglicht ein erleichtertes An- und Ausziehen der Kompressionshose sowie eine erhöhte Flexibilität in der Höhenverstellung bei verschiedenen Körperumfängen einer zu behandelnden Person und eine effiziente Druckanpassung auf den Unterbauch, um ein Ödem effektiv behandeln zu können. Die flächig ausgebildeten ersten Befestigungsmittel an der Vorderseite des Taillenbands, die bei angelegtem Taillenband im Bauchbereich des Patienten zu liegen kommen, ermöglichen dabei ein präzises Einstellen des Kompressionsdrucks auf den Genital- und Leistenbereich des Patienten. Ein Ödem im Genital- oder Leistenbereich eines Patienten kann dabei von dem als einteiliger Beutel oder als einteilige Schürze aus einem elastischen Textilmaterial ausgebildeten Kompressionselement vollständig umfasst werden, um einen gleichmäßigen sowie individuell einstellbaren Kompressionsdruck auf das Ödem auszuüben. Dabei wird durch die flächige Ausbildung des Kompressionselements ein Einschneiden in die empfindlichen Weichteile des Patienten vermieden, wodurch der Tragekomfort verbessert wird.

Ein erstes Ende des Kompressionselements ist dabei bspw. mit einer nicht lösbaren Verbindung, insbesondere mit einer Naht, an dem Hosenteil befestigt. Die nicht lösbare Verbindung ist dabei insbesondere in einem Schrittbereich, welcher bei angezogenem Hosenteil im Schritt des Patienten zu liegen kommt, an einer Innenseite des Hosenteils angeordnet. In einer alternativen Ausführungsform kann das Kompressionselement auch mit einer lösbaren Verbindung, bspw. einem Klettverschluss oder mittels Druckknöpfen, an der Innenseite des Hosenteils befestigt sein. Die lösbare Verbindung befindet sich ebenfalls im Schrittbereich des Hosenteils. Eine lösbare Verbindung des Kompressionselements an dem Hosenteil hat den Vorteil, dass das Kompressionselement ausgetauscht werden kann. Dies kann bspw. vorteilhaft sein, wenn in einer Behandlungsphase der Kompressionstherapie ein anderer Druckbereich durch das Kompressionselement erzeugt werden soll. Hierfür kann dann bspw. ein erstes Kompressionselement mit einer definierten Dehnbarkeit durch ein zweites Kompressionselement mit einer anderen Dehnbarkeit ersetzt werden.

An einem zweiten Ende des Kompressionselements, das dem ersten Ende gegenüberliegt, sind die zweiten Befestigungsmittel, die insbesondere als Klettband ausgebildet sein können, angeordnet. Bevorzugt sind die zweiten Befestigungsmittel als Klettband mit Widerhaken ausgebildet. Dies ermöglicht eine leichte und schnelle Fixierung der Kopplung der ersten und zweiten Befestigungsmittel an einer passenden Fixierposition sowie eine einstellbare Dehnung und damit einen einstellbaren Kompressionsdruck des Kompressionselements beim Anlegen der Kompressionshose an einem Patienten.

Bei an einem Patienten angelegter Kompressionshose ist das insbesondere aus einem elastischen Textilmaterial gebildete Kompressionselement mit einer von der Position der Kopplung der ersten und der zweiten Befestigungsmittel abhängigen Dehnung lösbar an dem Taillenband fixiert und übt aufgrund der Dehnung einen vom Dehnungsgrad abhängigen Kompressionsdruck auf den Genitalbereich und die Leistengegend des Patienten aus. Der Kompressionsdruck ist dabei durch Auswahl einer Kopplungsposition, an der die zweiten Befestigungsmittel mit den ersten Befestigungsmitteln gekoppelt sind, einstellbar. Der ausgeübte Kompressionsdruck kann dabei auch nach Anlegen der Kompressionshose am Patienten noch geändert werden, um bspw. den Kompressionsdruck im Verlauf einer längeren Behandlungsperiode anpassen zu können. Zur Anpassung des Kompressionsdrucks ist dabei nur eine Änderung der Kopplungsposition der ersten und der zweiten Befestigungsmittel erforderlich, ohne dass die gesamte Kompressionshose abgenommen werden muss.

Vorteilhaft in Bezug auf ein bequemes Tragen der Kompressionshose ist eine bereichsweise Überlappung eines oberen Abschnitts des Hosenteils, insbesondere eines Bundabschnitts des Hosenteils, mit dem darunter angeordneten Taillenband, wenn die Kompressionshose am Patienten angelegt ist. Es ist jedoch möglich, dass das Hosenteil vollständig unterhalb und im Abstand zum Taillenband am Patienten angeordnet wird.

In einer bevorzugte Ausführungsform, bei der ein Bundbereich des Hosenteils mit dem Taillenband bereichsweise überlappt, sind an einer Innenseite des Hosenteils, insbesondere im Bundbereich, lösbare Befestigungselemente, insbesondere in Form einer Mehrzahl von Hakenteilen eines Klettverschlusses, angeordnet, mit denen das Hosenteil auf einer Außenseite des darunterliegenden Taillenbands abnehmbar befestigt werden kann. Dadurch ergibt sich eine zweiteilige Ausführungsform der Kompressionshose, bei der das Hosenteil und das Taillenband als voneinander trennbare Teile ausgebildet sind, da die Verbindung des Hosenteils und des Taillenbands über die lösbar ausgebildeten Befestigungselemente zum leichteren Aus- und Anziehen der Kompressionshose voneinander getrennt werden können. Unter trennbar wird dabei eine manuell ohne Hilfsmittel lösbare Trennung des Hosenteils vom Taillenband, insbesondere durch ein Lösen der lösbaren Befestigungselemente, mit denen das Hosenteil insbesondere am Bund mit dem Taillenband befestigt wird, verstanden.

Zur Verbesserung des Tragekomforts der Kompressionshose können zwei seitliche Längskanten des Kompressionselements zumindest teilweise und insbesondere in einem oberen Abschnitt des Kompressionselements nach innen eingeschlagen sein. Dies verbessert auch das vollständige Umfassen eines Ödems durch das Kompressionselement. Weiterhin können zur Verbesserung des Sitzes der Kompressionshose am Patienten an dem Hosenteil und/oder am Taillenband Hosenträger angeordnet sein. Zur stabilen Fixierung des Kompressionselements in einer gewünschten Trageposition im Genitalbereich des Patienten können weiterhin an dem Kompressionselement, insbesondere im Bereich der seitlichen Längskanten des Kompressionselements, zusätzliche Befestigungsbänder angeordnet sein.

Das Hosenteil der Kompressionshose ist bevorzugt ein Kompressionstextil aus einem elastischen Textilmaterial und ist insbesondere aus einem elastischen Kompressionsgestrick hergestellt, wobei das Textilmaterial bevorzugt so ausgewählt und das Hosenteil so dimensioniert ist, dass das Hosenteil, insbesondere im Hüft- und Beckenbereich, einen vorgegebenen Kompressionsdruck auf den Patienten ausübt. Besonders bevorzugt ist das Hosenteil aus einem elastischen Kompressionsgestrick mit einem Grundgestrick und einem darin eingelegten elastischen Schussfaden gefertigt. Dies ermöglicht die Einstellung eines gewünschten Kompressionsdrucks über die Auswahl und die Dehnung des Schussfadenmaterials und die Anpassung der Dimensionierung des Hosenteils auf die Anatomie des Patienten, insbesondere durch eine Maßanfertigung. Durch die Ausbildung des Hosenteils als ein Kompressionstextil wird zusätzlich zu dem vom Kompressionselement auf den Genitalbereich des Patienten ausgeübten Druck auch auf den Hüftbereich und/oder den Beckenbereich des Patienten ein vollumfänglich wirkender Kompressionsdruck erzeugt. Dadurch können Ödeme, die sich vom Genitalbereich über die Leistengegend bis in den Hüft- und Beckenbereich erstrecken, wirksam durch eine Kompressionstherapie behandelt werden.

Sowohl das Hosenteil als auch das Kompressionselement sind zweckmäßig jeweils aus einem elastischen Textilmaterial gefertigt. Bevorzugt ist das Hosenteil aus einem Kompressionsgestrick oder einem elastisch gewebten Textilmaterial gebildet. Auch das Kompressionselement kann aus einem Kompressionsgestrick oder einem gewebten Textilmaterial gebildet sein. Das Taillenband ist bevorzugt aus einem beidseitig mit einem Velourmaterial kaschierten Schaumstoff gebildet. Die Dehnbarkeit des Taillenbands ist bevorzugt geringer als die Dehnbarkeit des Hosenteils und des Kompressionselements.

In einer bevorzugte Ausführungsform ist eine an der Innenseite des Kompressionselements, die bei angelegter Kompressionshose zum Körper des Patienten weist, einlegbare und an das Kompressionselement lösbar, bspw. über einen Klettverschluss, anbringbare Pelotte vorgesehen, die zur Erhöhung des vom Kompressionselement auf ein Ödem ausgeübten Drucks dient und zweckmäßig eine an die Körperform der zu behandelnden Person angepasste Form hat. Insbesondere können hierbei Pelotten mit einer für Männer oder Frauen geeigneten Passform im Schritt- bzw. Dammbereich eingesetzt werden. Ergänzend oder anstelle einer Befestigung der Pelotte an der Innenseite des Kompressionselements mittels eines lösbaren Befestigungsmittels, wie z.B. eines Klettverschlusses, kann an dem Kompressionselement, insbesondere an seiner Innenseite, auch eine Tasche vorgesehen sein, in die die Pelotte herausnehmbar eingelegt werden kann.

Diese und weitere Vorteile sowie bevorzugte Merkmale der Erfindung ergeben sich aus dem nachfolgend unter Bezugnahme auf die Zeichnungen beschriebenen Ausführungsbeispiel.

Dabei zeigen
- **Fig. 1:**: Darstellung einer Kompressionshose mit einem Taillenband und einem Hosenteil mit integriertem Kompressionselement in einer Vorderansicht in einem an einem Patienten angelegten Zustand;
- **Fig. 2**:: Darstellung der Kompressionshose von Figur 1 in einem am Patienten angelegten Zustand in einer Rückansicht;
- **Fig. 3:**: Darstellung der Kompressionshose von Figur 1 in einer Vorderansicht mit heruntergezogenem Hosenteil zur besseren Darstellung des Kompressionselements und des Taillenbands der Kompressionshose;
- **Fig. 4:**: Darstellung der Kompressionshose von Figur 3 mit heruntergezogenem Hosenteil in einer Rückansicht;
- **Fig. 5:**: Darstellung der Kompressionshose von Figur 1 in einer Vorderansicht mit heruntergezogenem Hosenteil und aufgeklapptem Kompressionselement zur besseren Darstellung der Innenseite des Kompressionselements und des Taillenbands der Kompressionshose;
- **Fig. 6:**: Detailansicht der Innenseite des Hosenteils der Kompressionshose mit dem im Schrittbereich des Hosenteils daran befestigten Kompressionselement, wobei die Innenseite des Kompressionselements gezeigt ist;
- **Fig. 7**:: Detaildarstellung der Innenseite des Hosenteils der Kompressionshose von Figur 1 mit dem im Schrittbereich des Hosenteils daran befestigten Kompressionselement, wobei die Außenseite des Kompressionselements gezeigt ist;
- **Fig. 8:**: Detaildarstellung des Hosenteils der Kompressionshose von Figur 1 mit dem daran im Schrittbereich des Hosenteils befestigten Kompressionselement in einer Rückansicht;
- **Fig. 9:**: Detailansicht des in Figur 8 strichpunktiert dargestellten Bereichs, der die Innenseite des Kompressionselements zeigt.

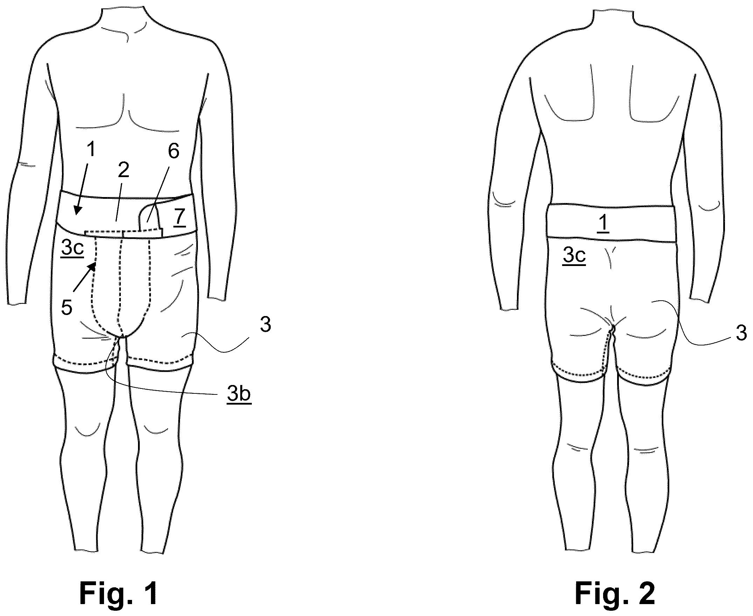

Die in den Figuren 1 und 2 in einer Vorderansicht und einer Rückansicht in einem an einem Patienten angelegten Zustand dargestellte Kompressionshose, welche insbesondere zur kompressiven Behandlung von Ödemen oder anderer Erkrankungen oder Verletzungen im Genital- oder Beckenbereich eines Patienten eingesetzt werden kann, umfasst ein Taillenband 1, ein davon getrenntes Hosenteil 3 und ein an dem Hosenteil befestigtes und an der Innenseite des Hosenteils 3 angeordnetes Kompressionselement 5, welches in der Darstellung von Figur 1 gestrichelt gezeigt ist, da es vom Hosenteil 3 überdeckt wird.

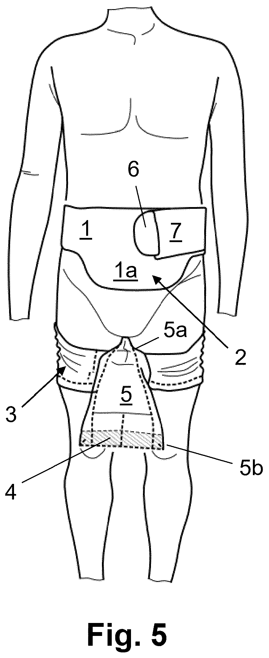

Das Taillenband 1, welches insbesondere der Darstellung von Figur 5 im Detail entnommen werden kann, ist als Band, welches im angelegten Zustand die Taille des Patienten vollumfänglich umgibt, ausgebildet und enthält einen zentralen Abschnitt 1a, der im Vergleich zu den sich zu beiden Seiten des zentralen Abschnitts 1a nach außen anschließenden Randabschnitten nach unten (also in distaler Richtung) verlängert ist. Der zentrale Abschnitt 1a, der bei am Patienten angelegten Taillenband 1 im Bauchbereich des Patienten zu liegen kommt, ist als flächiges Flauschteil eines Klettverschlusses ausgebildet und bildet dadurch erste Befestigungsmittel 2.

An einem Ende 7 des Taillenbands 1 sind Verschlusselemente 6 angeordnet. Bei diesen Verschlusselementen 6 kann es sich beispielsweise um ein Klettelement mit Wiederhaken eines Klettverschlusses oder um eine Gürtelschnalle handeln. Das Taillenband 1 kann mittels der Verschlusselemente 6 im Bereich der Taille des Patienten durch Kopplung der Verschlusselemente 6, beispielsweise durch Festlegen eines Klettverschlusses oder durch Schließen einer Gürtelschnalle, fixiert werden. Durch die Positionierung der Fixierungsstelle der Verschlusselemente 6 kann dabei ein ausgewählter Druck, der vom Taillenband 1 auf die Taille des Patienten ausgeübt wird, eingestellt werden. Zum Abnehmen des Taillenbands 1 vom Patienten können die Verschlusselemente 6 gelöst werden, indem beispielsweise die Kopplung des Klettverschlusses gelöst oder die Gürtelschnalle geöffnet wird.

Unterhalb des Taillenbands 1 befindet sich bei am Patienten angelegter Kompressionshose in teilweiser Überlappung mit dem darunterliegenden Taillenband 1 das Hosenteil 3 der Kompressionshose. Das Hosenteil 3 ist in dem zeichnerisch dargestellten Ausführungsbeispiel als Short mit einem schlauchförmigen Rumpfabschnitt 3a, der mittig einen Schrittbereich 3b enthält, zwei am Rumpfabschnitt 3a angeordnete schlauchförmige Beinabschnitte 3c und einem am oberen Rand des Rumpfabschnitts 3a angeordneten Bund 3d ausgebildet. Die Länge der beiden Beinabschnitte 3c ist dabei variabel, d.h. die Länge der Beinabschnitte 3c kann länger oder kürzer sein als in dem hier zeichnerisch dargestellten Ausführungsbeispiel. Insbesondere kann das Hosenteil 3 auch als Strumpfhose mit Beinabschnitten 3c ausgebildet sein, wobei sich die Beinabschnitte bis zum Knöchel des Patienten erstrecken können und optional auch ein Fußteil enthalten können.

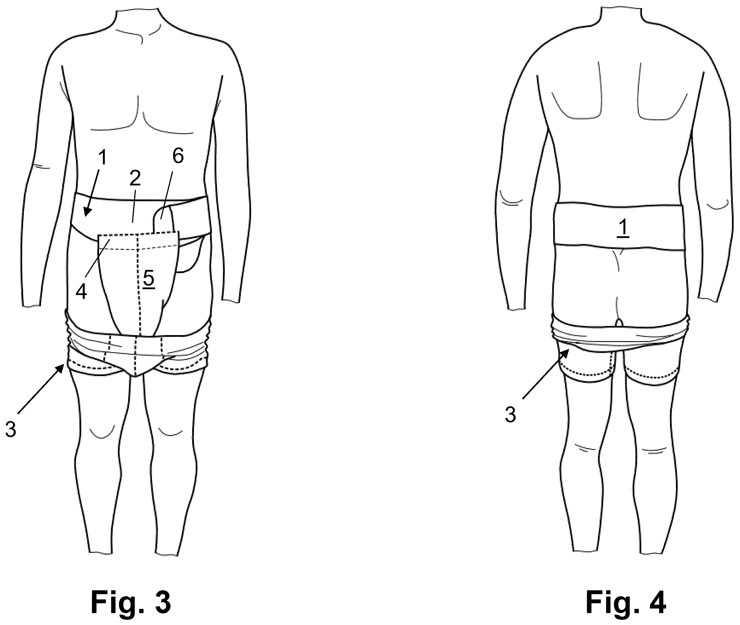

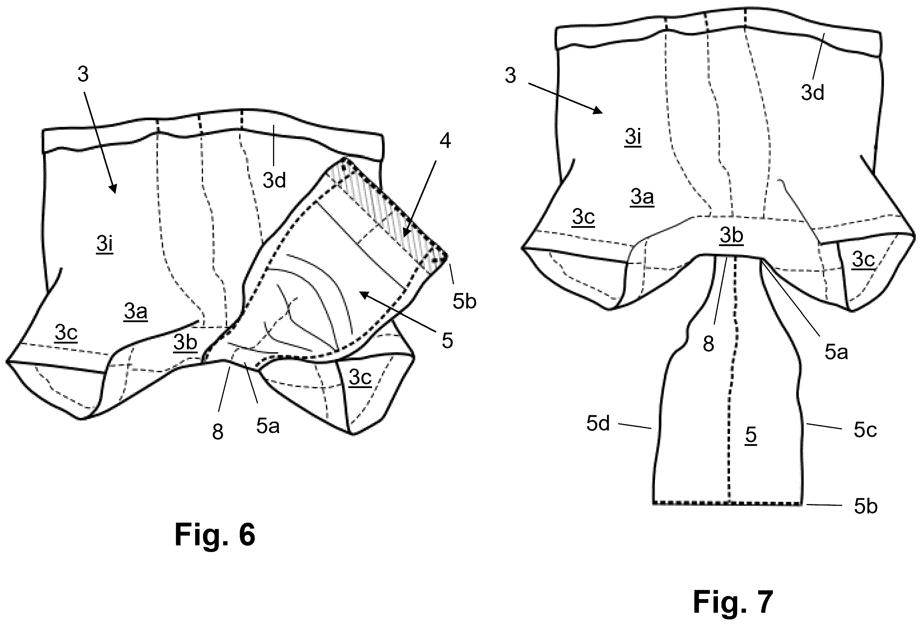

An der Innenseite 3i des Hosenteils 3, die in den Figuren 6 und 7 zu sehen ist, ist im Schrittbereich 3b ein Kompressionselement 5 befestigt. Das insbesondere aus den Figuren 3, 5 sowie 6 bis 9 ersichtliche Kompressionselement 5 ist als einteiliger Beutel oder als einteilige Schürze aus einem elastischen Textilmaterial gebildet. Das Kompressionselement 5 weist dabei ein erstes Ende 5a und ein diesem gegenüberliegendes zweites Ende 5b und sich dazwischen erstreckende seitliche Längskanten 5c, 5d auf, wie aus Figur 7 ersichtlich. Das erste Ende 5a des Kompressionselements 5 ist dabei an der Innenseite 3i des Hosenteils 3 im Schrittbereich 3b mittels einer Naht 8 befestigt, wie aus den Figuren 5 bis 9 ersichtlich. Am freien zweiten Ende 5b des Kompressionselements 5 sind zweite Befestigungsmittel 4 angeordnet. In dem zeichnerisch dargestellten Ausführungsbeispiel handelt es sich bei den zweiten Befestigungsmitteln 4 um ein Klettband, das im Bereich der oberen Kante am freien zweiten Ende 5b des Kompressionselements 5 angeordnet ist. Die zweiten Befestigungsmittel 4 dienen zur lösbaren Befestigung des Kompressionselements 5 an den ersten Befestigungsmitteln 2, die am vorderen Zentralabschnitt 1a des Taillenbands 1 vorgesehen sind. Bevorzugt sind die zweiten Befestigungsmittel 4 als ein Klettband mit Wiederhaken ausgebildet, die mit einem im vorderen Zentralabschnitt 1a des Taillenbands 1 angeordneten Klettpad (Flauschteil) eines Klettverschlusses zusammenwirken. Durch eine Kopplung der zweiten Befestigungsmittel 4 des Kompressionselements 5 mit den ersten Befestigungsmitteln 2 des Taillenbands 1 kann das freie, zweite Ende 5b des Kompressionselements 5 lösbar an einer ausgewählten Position am Taillenband 1 fixiert werden. Die Fixierung des freien zweiten Endes 5b des Kompressionselements 5 am vorderen Zentralabschnitt 1a des Taillenbands 1 ist beispielsweise der Darstellung von Figur 3 zu entnehmen. In Figur 5 ist das Kompressionselement 5 in einem vom Taillenband 1 abgenommenen Zustand gezeigt, wobei die Innenseite des Kompressionselements 5, an der die zweiten Befestigungsmittel 4 im Bereich des freien zweiten Endes 5b angeordnet sind, zu sehen ist.

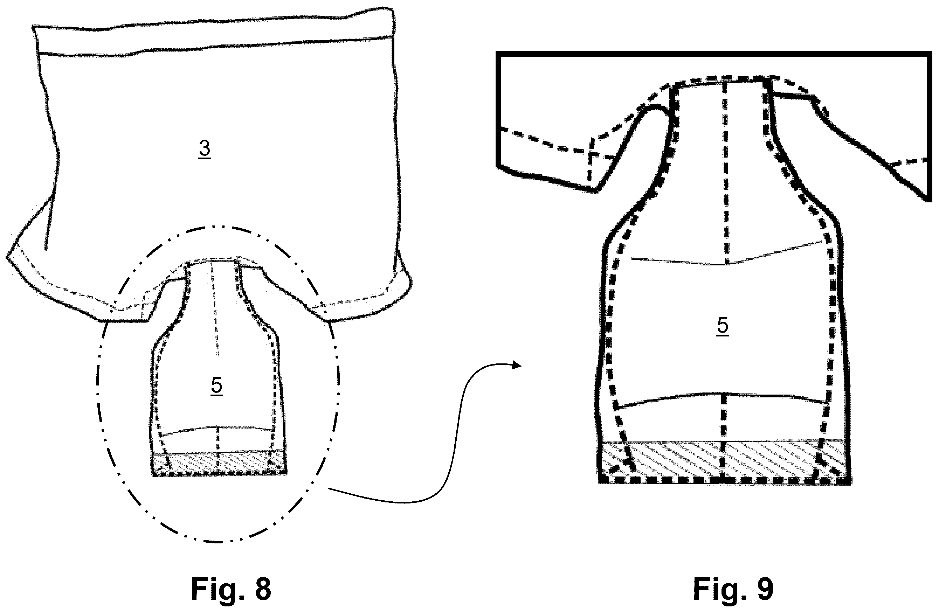

Die Innenseite des Kompressionselements 5 mit den dort am freien, zweiten Ende 5b angeordneten zweiten Befestigungsmitteln 4 ist auch aus Figur 6 ersichtlich. In Figur 7 ist die Außenseite des Kompressionselements 5 mit den beiden seitlichen Längskanten 5c, 5d gezeigt. Die Figuren 8 und 9 zeigen die Innenseite des Kompressionselements 5 in einer Detailansicht. Aus den Figuren 8 und 9 ist ersichtlich, dass die beiden seitlichen Längskanten 5c, 5d des Kompressionselements 5 in einem Abschnitt im Bereich des freien zweiten Endes 5b nach innen, d.h. zur Innenseite hin, eingeschlagen sind. Die eingeschlagenen seitlichen Längskanten sind dabei mit einer Naht fixiert und in den Figuren 8 und 9 gestrichelt dargestellt.

Zum Anlegen der Kompressionshose an einem Patienten wird zunächst das Taillenband 1 um die Taille des Patienten gelegt und mittels der Verschlusselemente 6 fixiert. Durch Wahl der Verschlussposition der Verschlusselemente 6 kann dabei ein bestimmter Druck vom Taillenband 1 auf den Taillen- und Hüftbereich des Patienten ausgeübt werden.

Nach Anlegen des Taillenbands 1 zieht der Patient das Hosenteil 3 an, wobei das im Schrittbereich 3b des Hosenteils 3 angenähte Kompressionselement 5 an der Innenseite 3i des Hosenteils 3 zu liegen kommt. Das freie, zweite Ende 5b des Kompressionselements 5 wird danach an der Oberseite des Hosenteils 3, insbesondere im Bereich des Bunds 3d aus dem Hosenteil 3 herausgezogen, wie in Figur 1 durch die gestrichelten Linien des Kompressionselements 5 angedeutet. Mittels der zweiten Befestigungsmittel 4, die an der Innenseite des Kompressionselements 5 im Bereich des freien, zweiten Endes 5b angeordnet sind, wird das Kompressionselement 5 durch Kopplung der zweiten Befestigungsmittel 4 mit den ersten Befestigungsmitteln 2 am Taillenband 1 fixiert. Aufgrund der elastischen Eigenschaften des Kompressionselements, welches aus einem elastischen Textilmaterial gebildet ist, wird das Kompressionselement 5 je nach Fixierungsposition der ersten und der zweiten Befestigungsmittel 2, 4 mehr oder weniger gedehnt. Durch den Grad der Dehnung des Kompressionselements 5 kann ein Kompressionsdruck, der vom Kompressionselement 5 auf den Genitalbereich und die Leistengegend des Patienten ausgeübt wird, ausgewählt werden. Die Fixierposition, an der die zweiten Befestigungsmittel mit den flächig ausgebildeten ersten Befestigungsmitteln gekoppelt sind, legt damit den vom Kompressionselement 5 auf den Genitalbereich des Patienten ausgeübten Kompressionsdruck fest.

In einer bevorzugten Ausführungsform sind an der Innenseite 3i des Hosenteils 3 im Bereich des Bunds 3d zusätzliche Befestigungselemente 9, insbesondere in Form einer Mehrzahl von Hakenteilen eines Klettverschlusses oder Druckknöpfe, vorgesehen. Mit diesen zusätzlichen Befestigungselementen 9 kann die Innenseite 3i des Hosenteils 3, insbesondere im Bereich des Bunds 3d, auf der Außenseite des im Überlappungsbereich darunterliegenden Taillenbands 1 abnehmbar befestigt werden. Wenn die zusätzlichen Befestigungselemente 9 Druckknöpfe sind, enthält bspw. die Außenseite des Taillenbands entsprechend der Anzahl der Druckknöpfe eine Mehrzahl von korrespondierenden Aufnahmen mit Vertiefungen, in die jeweils ein an der Innenseite des Bunds des Hosenteils 3 angeordnetes Kopfteil eines Druckknopfs lösbar verankert werden kann, oder umgekehrt.

Zum Ausziehen der Kompressionshose wird der oben beschriebene Vorgang zum Anlegen der Kompressionshose an einem Patienten in umgekehrter Reihenfolge durchgeführt. Hierfür wird zunächst das Hosenteil 3, wie in Figur 3 und Figur 4 gezeigt, nach unten geschoben und anschließend kann die Kopplung der zweiten Befestigungsmittel 4 an den ersten Befestigungsmitteln 2 gelöst werden, um die Verbindung des freien, zweiten Endes 5b des Kompressionselements 5 mit dem Taillenband 1 zu lösen. Danach kann das Hosenteil mit dem integrierten Kompressionselement 5 ausgezogen und schließlich das Taillenband 1 vom Körper des Patienten entfernt werden.

Die Ausbildung der Kompressionshose in zweiteiliger Form mit dem Taillenband 1 und dem davon getrennten bzw. davon trennbaren Hosenteil 3 mit integriertem Kompressionselement 5 ermöglicht dabei ein einfaches und bequemes An- und Ausziehen der Kompressionshose. Beim Anziehen der Kompressionshose kann dabei durch Festlegung der Kopplungsposition, an der die zweiten Befestigungsmittel 4 an den flächigen ersten Befestigungsmitteln 2 gekoppelt sind, eine bestimmte Dehnung des elastischen Kompressionselements 5 eingestellt werden. Durch die Einstellung der Dehnung des Kompressionselements 5 wird der Kompressionsdruck, der vom Kompressionselement auf den Genitalbereich und die Leistengegend des Patienten ausgeübt wird, eingestellt. Der vom Kompressionselement 5 ausgeübte Kompressionsdruck kann dabei nachträglich, d.h. nach Anlegen der Kompressionshose am Patienten, noch nachjustiert werden. Dies ist beispielsweise bei einer länger anhaltenden Kompressionstherapie von Vorteil, um den Kompressionsdruck an den Heilungsverlauf anpassen zu können. So ist es z.B. möglich, den Kompressionsdruck, je nach Heilungsverlauf, schrittweise während der Behandlungsdauer zu verringern.

Sowohl das Hosenteil 3 als auch das Kompressionselement 5 sind zweckmäßig jeweils aus einem elastischen Textilmaterial gefertigt. Bevorzugt ist das Hosenteil aus einem Kompressionsgestrick mit einem Grundgestrick aus einem elastischen Faden und einem darin eingelegten bzw. eingestrickten elastischen Schussfaden gebildet. Insbesondere kann es sich bei dem Material des Hosenteils 3 um eine rundgestrickte Ware aus 80% Polyamid und 20% Elasthan handeln. Auch das Kompressionselement 5 kann aus einem Kompressionsgestrick gebildet sein. Das Kompressionselement 5 ist bevorzugt ein gewebtes Textilmaterial, beispielsweise aus 71% Polyamid und 31% Elasthan. Das Taillenband 1 ist bevorzugt aus einem beidseitig mit einem Velourmaterial kaschierten Schaumstoff gebildet, beispielsweise aus 74% Polyamid, 11% Polyurethan und 15% Elasthan. Die Dehnbarkeit des Taillenbands 1 ist dabei bevorzugt geringer als die Dehnbarkeit des Hosenteils 3 und des Kompressionselements 5.

## Patentansprüche

1. Kompressionshose, insbesondere zur kompressiven Behandlung von Ödemen oder anderer Erkrankungen oder Verletzungen im Genital- oder Beckenbereich eines Patienten, umfassend ein Taillenband (1) mit ersten Befestigungsmitteln (2), ein Hosenteil (3) und ein mit zweiten Befestigungsmitteln (4) ausgestattetes Kompressionselement (5) zur Ausübung einer Kompression auf den Genitalbereich des Patienten, wobei das Taillenband (1) mittels Verschlusselemente (6) verstellbar im Bereich der Taille oder im oberen Hüftbereich des Patienten anlegbar ist, wobei die ersten Befestigungsmittel (2) zumindest im oder unterhalb des Bauchbereich des Patienten zu liegen kommen, und das Hosenteil (3) im angezogenen Zustand zumindest den Beckenbereich des Patienten vollumfänglich umschließt und das Kompressionselement (5) mittels einer lösbaren Kopplung der ersten Befestigungsmittel (2) und der zweiten Befestigungsmittel (4) an dem Taillenband (1) befestigbar ist, **dadurch gekennzeichnet, dass** das Taillenband (1) und das Hosenteil (3) voneinander getrennte oder voneinander trennbare Teile der Kompressionshose sind und dass das Kompressionselement (5) als einteiliger Beutel oder als einteilige Schürze aus einem elastischen Textilmaterial ausgebildet und an dem Hosenteil (3) befestigt ist.

2. Kompressionshose nach Anspruch 1 oder 2, wobei das Kompressionselement (5) mit einer lösbaren Verbindung oder einer nicht lösbaren Verbindung, insbesondere mit einer Naht (8), an dem Hosenteil (3) befestigt ist, wobei die Verbindung insbesondere in einem Schrittbereich (3b), welcher bei angezogenem Hosenteil (3) im Schritt des Patienten zu liegen kommt, angeordnet ist.

3. Kompressionshose nach Anspruch 1 oder 2, wobei das Kompressionselement (5) ein erstes Ende (5a) und ein zweites Ende (5b) umfasst, wobei das erste Ende (5a) an dem Hosenteil (3), insbesondere in einem Schrittbereich (3b), welcher bei angezogenem Hosenteil (3) im Schritt des Patienten zu liegen kommt, befestigt und bevorzugt angenäht ist und die zweiten Befestigungsmittel (4) an dem zweiten Ende (5b) des Kompressionselements (5) angeordnet sind.

4. Kompressionshose nach Anspruch 3, wobei das erste Ende (5a) des Kompressionselements (5) an einer Innenseite (3i) des Hosenteils (3) befestigt ist und das zweite Ende (5b) des Kompressionselements (5) bei am Patienten angelegter Kompressionshose mittels der Kopplung der ersten Befestigungsmittel (2) und der zweiten Befestigungsmittel (4) lösbar an dem Taillenband (1) fixiert oder fixierbar ist.

5. Kompressionshose nach einem der voranstehenden Ansprüche, wobei das Kompressionselement (5) aus einem elastischen Material, insbesondere einem elastischen Textilmaterial, ist und bei am Patienten angelegter Kompressionshose unter Dehnung des elastischen Materials an dem Taillenband (1) befestigt werden kann, wodurch das Kompressionselement (5) einen Kompressionsdruck auf den Genitalbereich des Patienten ausübt, wobei der Kompressionsdruck durch Auswahl einer Kopplungsposition, an der die zweiten Befestigungsmittel (4) mit den ersten Befestigungsmitteln (2) gekoppelt sind, einstellbar ist.

6. Kompressionshose nach einem der voranstehenden Ansprüche, wobei das Hosenteil (3) vollständig unterhalb des Taillenbands (1) oder mit dem darunterliegenden Taillenband (1) zumindest teilweise überlappend angeordnet ist, wenn die Kompressionshose am Patienten angelegt ist.

7. Kompressionshose nach Anspruch 8, wobei das Hosenteil (3) mit dem darunterliegenden Taillenband (1) zumindest teilweise überlappend angeordnet ist, wenn die Kompressionshose am Patienten angelegt ist, wobei an einem oberen Abschnitt des Hosenteils (3), der mit dem darunter angeordneten Taillenband (1) überlappt, an einer Innenseite (3i) des Hosenteils (3) Befestigungselemente, insbesondere eine Mehrzahl von Hakenteilen eines Klettverschlusses, angeordnet sind, mit denen das Hosenteil (3) auf einer Außenseite des darunterliegenden Taillenbands (1) abnehmbar befestigt werden kann.

8. Kompressionshose nach einem der voranstehenden Ansprüche, wobei die ersten Befestigungsmittel (2) und die zweiten Befestigungsmittel (4) jeweils miteinander korrespondierende Bestandteile eines zweiteiligen, reversiblen Verbindungsmittels, insbesondere eines Klettverschlusses, eines Haftverschlusses oder einer Druckknopfverbindung, sind, wobei bevorzugt die ersten Befestigungsmittel (2) flächig und insbesondere als flächiges Flauschteil eines Klettverschlusses ausgebildet sind und auf einer Außenseite eines vorderen Abschnitts (1a) des Taillenbands (1), der bei angelegtem Taillenband (1) im Bauchbereich des Patienten zu liegen kommt, angeordnet sind.

9. Kompressionshose nach einem der voranstehenden Ansprüche, wobei das Kompressionselement (5) zwei seitliche Längskanten (5c, 5d) aufweist, wobei diese seitlichen Längskanten (5c, 5d) bevorzugt zumindest teilweise und insbesondere in einem Abschnitt (5') am freien Ende (5b) des Kompressionselements (5) nach innen eingeschlagen sind.

10. Kompressionshose nach einem der voranstehenden Ansprüche, wobei an dem Hosenteil (3) und/oder am Taillenband (1) Hosenträger angeordnet sind und/oder an dem Kompressionselement (5), insbesondere im Bereich der seitlichen Längskanten (5c, 5d) des Kompressionselements (5), Befestigungsbänder angeordnet sind.

11. Kompressionshose nach einem der voranstehenden Ansprüche, wobei das Hosenteil (3) ein Kompressionstextil aus einem elastischen Textilmaterial ist und insbesondere aus einem elastischen Kompressionsgestrick hergestellt ist, wobei das Textilmaterial bevorzugt so ausgewählt und das Hosenteil so dimensioniert ist, dass das Hosenteil (3), insbesondere im Hüft- und Beckenbereich, einen vorgegebenen Kompressionsdruck auf den Patienten ausübt.

12. Kompressionshose nach einem der voranstehenden Ansprüche, wobei die Verschlusselemente (6) als lösbare Befestigungselemente, insbesondere als Klettverschluss oder als Gürtelschnalle, ausgebildet und in einem Endabschnitt (7) des Taillenbands (1) angeordnet sind.

13. Kompressionshose nach einem der voranstehenden Ansprüche, wobei die Verschlusselemente (6), mit denen das Taillenband (1) lösbar am Patienten, insbesondere in dessen Bauchbereich, befestigbar ist, die Einstellung eines durch das Taillenband (1) auf den Taillenbereich und/oder den Hüftbereich des Patienten ausgeübten Drucks ermöglichen, indem die Verschlusselemente (6) in einer Umfangsrichtung des Patienten an unterschiedlichen Positionen fixierbar sind.

14. Kompressionshose nach einem der voranstehenden Ansprüche, **gekennzeichnet durch** eine am Kompressionselement (5) lösbar anbringbare oder in eine am Kompressionselement (5) angeformte Tasche einlegbare Pelotte.

15. Kompressionshose nach einem der voranstehenden Ansprüche, wobei das Kompressionselement (5) des Kompressionselements (5) bei am Patienten angelegter Kompressionshose innerhalb des Hosenteils (3) liegt.
